# EUROPEAN PATENT APPLICATION

(11) **EP 3 777 788 A1**
(43) Date of publication of application: **17.02.2021**
(21) Application number: 19777462.3
(22) Date of filing: 26.03.2019
(51) Int. Cl.: A61F 9/007, A61L 31/04

(54) **DRAINAGE DEVICE FOR THE SURGICAL TREATMENT OF GLAUCOMA**

(30) Priority: 26.03.2018 RU 2018110662
(71) Applicant: Korigodskiy, Aleksandr Robertovich, Moscow 107113 (RU); Zakharov, Ivan Dmitrievich, Moscow, 127055 (RU)
(72) Inventor: Korigodskiy, Aleksandr Robertovich, Moscow 107113 (RU); Zakharov, Ivan Dmitrievich, Moscow, 127055 (RU)
(74) Representative: AWA Denmark A/S
(86) International application number: PCT/RU2019/000187
(87) International publication number: WO 2019/190353

(57) **Abstract**

The utility model relates to medical technology, and more particularly to drainage devices for use in ophthalmic surgery in the case of penetrating glaucoma procedures. A drainage device for glaucoma surgery is configured in the form of a flat finished product made of a bioresorbable microfibre material and having a first end for at least partial insertion beneath a dissected scleral flap, and a second end configured such that it can be positioned in the scleral-conjunctival zone beneath the dissected conjunctiva, wherein the diameter of the fibres is in a range of 0.1-6.0 µm.

## Description

### TECHNICAL FIELD

The utility model relates to the medical equipment, namely to drainages used in ophthalmic surgery, and can be used in the surgical treatment of various types of glaucoma (primary, refractory, repeated surgical interventions, in complex cases with secondary glaucoma).

### STATE OF THE ART

Glaucoma is a large group of eye diseases with characteristic visual impairment. In many cases, refusal of surgery or its untimely performance leads to a progressive drop in visual impairment and blindness. Antiglaucomatous operations allow creating new outflow pathways of aqueous humor from the eye.

Intraocular fluid (IOF), or aqueous humor, is known to be an important source of nutrition for internal structures of the eye. It circulates mainly in the anterior segment of the eye. It participates in the metabolism of the lens, the cornea, the trabecular apparatus, the vitreous body and plays an important role in maintaining a certain level of intraocular pressure (IOP) [E.N. Iomdina, S.M. Bauer, K.E. Kotlyar. Biomechanics of the eye: theoretical aspects and clinical applications. M., Real Time, 2015, 208 p.].

Intraocular fluid is continuously produced by the branching of the ciliary body, accumulates in the posterior chamber. Then most of it flows through the pupil, washing the lens, then enters the anterior chamber and passes through the drainage system of the eye, located in the zone of an angle of the anterior chamber - the trabecula and the Schlemm canal. Aqueous humor bleeds through the trabecular meshwork and is collected in the Schlemm canal, which is a circular chink with a lumen diameter of about 0.3-0.5 mm, and then goes to the surface of the sclera, enters the sclero-conjunctival surface and from there flows through a system of thin tubules that flow into the episcleral (surface) veins of the eye, as well as the vasculature of the conjunctiva, in which IOF is intensively utilized and which are a final point of the outflow.

Tissue splicing in outflow zones of intraocular fluid (usually endogenous in nature or as a result of previous surgical interventions), regardless of the location, can lead to difficulty in its passage, a steady increase in intraocular pressure and, ultimately, is the main cause of glaucoma.

Antiglaucomatous operations aimed at creating new outflow pathways can be divided into two main groups:
1. Non-penetrating operations, during which the deep layer of corneoscleral tissues and the outer wall of the Schlemm canal are removed, as well as the epithelial layer of the inner wall of the Schlemm canal and the descemet's membrane is released. IOF filtration is carried out through the pores of the remaining trabecular meshwork and the descemet's membrane. The most widely used operation is non-penetrating deep sclerectomy, which minimizes the scope of intervention and the risk of post-surgery complications.
2. Penetrating (fistulizing) operations, during which the anterior chamber of the eye is opened with further formation in the limbal zone of the fistula, which is a part of the newly created outflow pathways of IOF. These operations, which primarily include trabeculectomy, are used in ophthalmic surgery due to the relative ease of performance and a persistent decrease in IOP.

In all antiglaucomatous operations, a provisional scleral flap (triangular, rectangular, or trapezoidal) is dissected, the base of which (2.0-4.0 mm in size) usually starts from the limb, and the top is 3-5 mm from the limb. Thus, usually the size of the scleral flap is 2.0-4.0*3.0-5.0 mm.

The use of drainages in the surgical intervention zone is the most effective way to preserve the outflow pathways of IOF (reducing scarring in the intervention area) created during antiglaucomatous operations. At the same time, the drainages used can be divided into non-resorbable and (bio)resorbable depending on the materials used for their production.

It is known that implants based on non-resorbable bioinert polymer materials are widely used in medicine for various surgical operations, including antiglaucomatous ones. However, most of these materials have significant disadvantages, in particular, a tendency to calcification, which begins with formation of hydroxyapatite crystals on the surface of the materials, if they are solid, and in volume, if they are permeable to water (porous or containing water with its structure) [Biocompatibility. Under the editorship of V.I. Sevastyanov. M., 1999, 368 p.]. In particular, these materials include polytetrafluoroethylene (teflon), polyethylene terephthalate (dacron), hydrogel materials, including polyhydroxyethylmethacrylate (poly-HEMA, cross-linked) and collagen (cross-linked). This disadvantage is compounded by the fact that non-resorbable materials and implants based on them, in particular, antiglaucomatous drainages, are intended for permanent residence in the body, and over time, the processes of calcification of implants increase.

A non-resorbable hydrogel T-shaped drainage is known to be intended for minimally invasive non-penetrating antiglaucomatous operations [A.V. Tereshchenko, I.A. Molotkova, Yu.A. Bely, E.V. Erokhina. Modification of modern microinvasive non-penetrating glaucoma surgery with the use of the T-shaped drainage. Ophthalmosurgery. 2011, Nº2 [http://www.eyepress.ru/article.aspx?9402]. The T-shaped drainage cut from cross-linked hydrogel polyacrylate material (NEP "Eye microsurgery") has the following characteristics: a length - 2.5 mm, a thickness - 0.3 mm, sizes of the horizontal part - 2.0*0.5 mm, vertical - 1.5*1.0 mm. The resulting drainage was placed in the formed scleral bed and not fixed. The drainage was retained in the bed by suture fixation to the sclera layers of the surface scleral flap, which was moved in place. It is noted that the drainage is elastic, has a high humor permeability, and is not subject to biodegradation and swelling. Dense filling of the dissected intrascleral cavity with a hydrogel implant contributes to the long-term prevention of fibroblastic scarring processes in the operation zone. However, this drainage, placed in the scleral bed, does not prevent formation of sclero-conjunctival splices, and may also be subject to calcification during long periods of implantation, inevitably leading to undesirable hypertension.

A non-resorbable drainage is known to be intended for performing penetrating antiglaucomatous operations in the form of a device that includes a body made of a polymer material based on a hydrogel containing 70-76 % water in the polymer [RF Patent Nº2192821, 2002. A method of surgical treatment of severe forms of glaucoma. V.I.. Sevastyanov, A.M. Immortal, V.P. Erichev at al]. At the same time, the drainage body is provided with 2-3 cylindrical microtubules protruding above the surface of the body, made of a microporous polymer compound, and during implantation these microtubules are placed in the anterior chamber, and the drainage body is fixed intrasclerally (under the scleral flap). The disadvantage of this drainage is an inevitable decrease in the filtration of the chamber humor through the pores of microtubules due to their clogging and chocking, as well as formation of sclero-conjunctival splices and, as a result, rise of IOP.

There is also a non-resorbable collagen antiglaucomatous drainage "Xenoplast", made from bone insoluble collagen of type 1 of animal origin (xenotex), which was used in non-penetrating and penetrating glaucoma surgery [S.Yu. Anisimova, S.I. Anisimov, E.V. Larionov at al. Antiglaucomatous "Xenoplast" drainage in non-penetrating and penetrating glaucoma surgery. X Congress of ophthalmologists of Russia. Collection of scientific materials. M., 2015, p. 71]. The drainage has a porous structure (corresponding to the original native spongy bone), sizes of 4.0*1.5*0.8 mm. The shape and the size of the implant can be modeled depending on the scope of surgery intervention. The porous structure of the drainage provides active absorption of intraocular fluid. The drainage is placed in the scleral bed and does not resorb after implantation for at least several years [S.Yu. Anisimova, S.I. Anisimov, I.V. Rogacheva, Surgical treatment of refractory glaucoma using a new, biodegradable collagen drainage. Glaucoma. 2006, Nº2, 51-56]. However, xenogenic tissue implants containing collagen are extremely susceptible to calcification, especially during long-term implantation, which can lead to the loss of main functional properties of these products [Biocompatibility. Under the editorship of V.I. Sevastyanov. M., 1999, 368 p.].

The closest to the application prototype drainage is a bioresorbable drainage intended for non-penetrating antiglaucomatous operations and is a device in the form of a hollow profile with a closed contour in cross-section with an ability of through-passing the scleral flap through the device cavity during surgical treatment [RF patent for utility model Nº126931, 2013. Drainage device. A.R. Korigodski, I.B. Alekseev, S.S. Long, A.J., Slonimsky]. The drainage is a flat product (article) and has flat walls, that allow the scleral flap to close closely (after its fixation) and the osculant section of the sclera, which prevents unwanted hypotension. When used, it prevents formation of splices under the scleral bed (sclero-scleral) and between the scleral flap and the conjunctiva, and also increases convenience and reliability of fixing the device on the scleral flap. However, during implantation, this drainage enters only the prilimbal zone of the sclero-conjunctival area, not reaching the zone of the active vascular outflow of IOF, which may reduce effectiveness of its action.

### SUMMARY OF THE TECHNICAL SOLUTION

The purpose of this application is to create a bioresorbable drainage intended for all types of antiglaucomatous operations, using which a long-term hypotensive effect is achieved while minimizing side reactions and complications.

Taking into account the above mentioned, there is a task to create a drainage for antiglaucomatous surgical operations, the use of which would allow for the smooth passage of IOF (stable outflow pathways) throughout the formed outflow zone, up to the conjunctival vasculature and prevent their splicing. Another task is to create a drainage that ensures creation and maintenance of an additional sclero-conjunctival cavity, especially in the zone of the active vascular outflow, which significantly increases the area of filtration of IOF into the capillarity and provides a long-term hypotensive effect. Another task is to create a universal drainage that could be used for various types and variants of antiglaucomatous operations.

In accordance with this application, the drainage for antiglaucomatous surgical operations is made in the form of a final flat product made of a bioresorbable material, having a first end (proximal) section (end) for at least partial insertion under the separated scleral flap and a second (distal) end section (end) for placement in the sclero-conjunctival zone under the separated conjunctiva, preferably in the zone of the active vascular outflow.

The application drainage should be a flat product, i.e. one in which longitudinal sizes (a length and a width) significantly exceed a thickness, since the drainage made of a flat material (with a thickness of no more than the above value) allows the scleral flap to close closely after its fixation and the osculant section of the sclera, which creates favorable conditions for the passage of IOF and proper tissue splicing. This is also facilitated by the soft elastic structure of most of the application drainages, especially microfiber ones. The application flat drainages can be bent as shown below, that, however, does not thicken their part exiting from under the scleral flap.

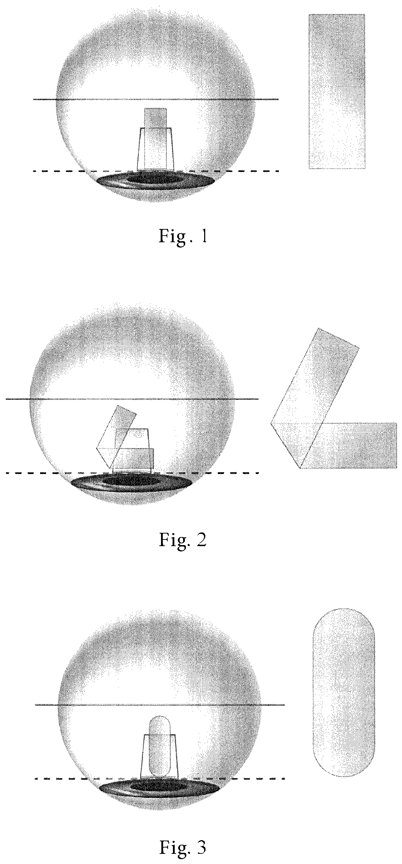

The drainage can be made in the form of a simple geometric shape - a strip (advantageously), a triangle, a square, a circle, and can also have a complex shape, for example, its distal part consists of several separate elements - ledges (Fig. 1-15).

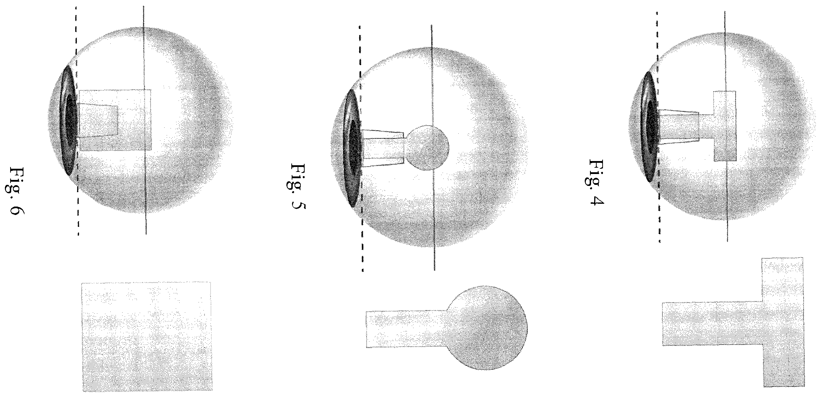

At the same time, its proximal section located under the scleral flap may be smaller than the scleral flap (placed under it) (Fig. 1-5, 7, 15) or larger than it (partially protruding from under it) (Fig. 6, 8-14).

As known, the episclera (a vascular sclero-conjunctival zone - a thin layer of the connective tissue between the conjunctiva and the sclera), starting from the limb (edge of the cornea) and ending at the posterior pole of the eye, is permeated with blood vessels, and the main vorticose veins (4-6 in number), in the zone which the most active vascular outflow of IOF occurs, are located in the equator (eyeball), 10-12 mm away from the limb, and 2.5-3.5 mm behind the equator [http://www.zdorlife.ru/page-id-512.html; E.A. Egorov, S.N. Basinsky. Clinical lectures on ophthalmology. Training guide. Geotar-Media. 2007, 163 p.]. A capillarity extends from these vessels towards the limb, the thickness of which to the periphery is monotonously reduced. Therefore, in the healthy eye, the vascular outflow is carried out throughout the episclera, but to the least extent - in the prilimbal area.

In glaucoma, due to the processes of spasm and sclerosis, venous capillaries, especially thin ones which are osculant to the limb, lose their draining ability. Therefore, the zone of the vascular outflow of patients with glaucoma is even more shifted from the limb to the equator. For this reason, only starting from the distance of 5-6 mm and, especially, at the distance of 10-12 mm, making pockets between the sclera and the conjunctiva (separating the conjunctiva) and insertion of the distal section of the antiglaucomatous drainage into this space is most effective. At the same time, since the edge of the scleral flap recedes from the limb by 4-6 mm, it is necessary the length of the distal drainage section (which protrudes beyond the scleral bed) to be at least 1-6 mm, advantageously 6-12 mm, that ensures the most active outflow of IOF.

Taking into consideration the above, the second (distal) section located under the scleral flap should have sizes that contribute to the greatest filtration (outflow) of IOF into the capillary system, and advantageously should have apertures (pores, perforations, individual large apertures) that contribute to this process. These apertures should advantageously be maximum, but not at the expense of supporting functions of the drainage (retention of the arch of the outflow channels) and its physical integrity.

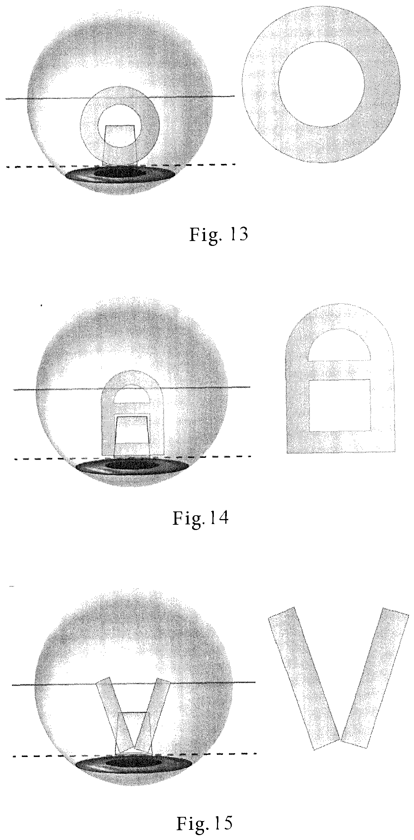

In addition, an increase in the area of the distal section will contribute to the greatest filtration of IOF. At the same time, with the same area of the second drainage section, the option with a longer length rather than a width is preferable, since, as indicated above, in the scleral zone of the vascular outflow, when approaching the equator, a density of absorbing capillaries capable of utilizing IOF increases. Therefore, increasing the length of the second section of the drainage and presence of not one, but several such extended sections will increase effectiveness of the drainage. In addition, the drainage may consist of several (two or more) identical or different elements installed independently of each other, for example, strips, that is preferable (Fig. 15). Also the drainage, for example, in the form of a strip, can be additionally bent in at least one line, which can also increase the surface of the outflow pathways of IOF and reach the zone of the active outflow (Fig. 2).

The drainages described above can be used for non-penetrating operations in accordance with this application.

Drainages intended for penetrating operations, in accordance with this application, additionally on the first end (proximal) section they have a ledge that faces mainly in the opposite direction from the second end (distal) section. This ledge during the penetrating operation should be able to be inserted into the channel formed in the zone of trabeculectomy, before its edge exits into the anterior chamber (to prevent splicing this channel). To solve this problem, the ledge must meet the following requirements:
- have a length not less than a certain value in order for his end to enter the front chamber, i.e., its size would not be less than the formed channel (to avoid splicing) and would not be more (than 0.2-1.0 mm) to avoid subsequent contact with non-resorbable (excess) of the end section of the ledge into the anterior chamber in the process of resorption of the entire drainage
- have a width (a size of the base) of at least a certain value so that the ledge can be conveniently inserted into the formed channel (so that the ledge is sufficiently rigid) and no more than a certain value so that its sizes allow it to be inserted into the specified channel
- have a thickness (of wall) of at least a certain value so that the ledge is sufficiently rigid and can be inserted into the formed channel without its deformation (bends)

Since it is preferable that the end part (edge) of the ledge protrudes slightly into the anterior chamber after implantation, it is necessary it to be resorbed faster in order to avoid getting into it not fully resorbed parts of the ledge-denticle. It is provided in the application drainages due to the shape of the end part of the ledge, preferably having a necking (at the end proximal part).

The ledge can have a shape selected from the following group: a triangle, a rectangle, or a trapezoid, or these shapes with rounded or beveled angels, or a semi-ellipse, or a shape of a thread, a loop, or connected threads, but is not limited to these shapes. This ledge can be explicitly or implicitly expressed (so that it cannot be explicitly isolated), being an extension of the drainage base (if the drainage has a shape of a triangle, a square, or a parallelogram).

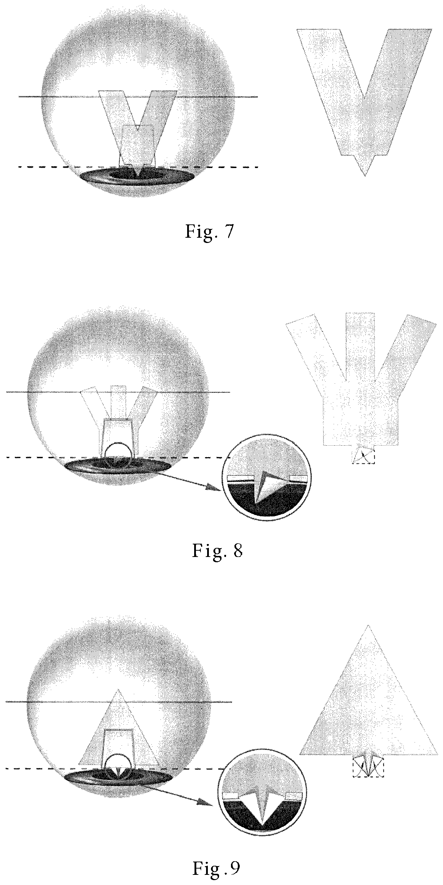

Besides, the ledge can be additionally bent in at least one line at a sharp angle to its base (Fig. 8 and 9). This increases rigidity of the inserted ledge in the trabeculectomy zone without unnecessary increasing the drainage wall thickness (and, consequently, the resorption time).

It should be noted that a thickness of the drainage in an unbent form is equal to a thickness of its walls, or, conventionally, a thickness of the original film-blank (see below), and a thickness of the drainage in a bent form (can be bent as the base of the drainage and the ledge, as shown above) is equal to a twice thickness of its walls. In any case, the total drainage thickness should not exceed the value specified below.

The sizes of the application drainage are in the following range: a width is 0.5-12.0 mm, a thickness (total) is 10-600 microns, a height is (total) 3-20 mm.

The drainage according to this application is made of a bioresorbable material, which allows to avoid side reactions and complications that are aggravated by long or permanent implantation periods. The application drainage, having fulfilled its temporary function of stabilizing IOP in the post-surgery period (due to mechanical maintenance of the arches and prevention of splicing the outflow pathways formed during the surgery intervention), completely resolves within 1-12 months, the optimal time is 2-5 months (that is necessary for formation the stable outflow pathways from tissues).

Synthetic polymers, microbiologically synthesized polymers, and modified natural polymers can be used as bioresorbable materials for drainage production.

The drainage can be made of a continuous material (film) or a non-continuous material: a porous film, at least a partially perforated film, as well as a micro-wave or a woven material or a mesh.

According to the following aspects of this application, there are methods for obtaining the drainage described above.

In accordance with the first method, a blank is first obtained, which is a flat sheet made of a bioresorbable material from a continuous, porous or perforated film, of a micro-wave or a woven material or a mesh. These blanks can be obtained by casting from a solution or a melt, or a woven from threads or fibers, as shown below. The finished products of an appropriate size and a shape are cut out of these sheets.

In accordance with the second method, a blank is first obtained, which is a thin-walled tube made of a bioresorbable material. Then the resulting blank is cut out in a flat folded or cut (for example, lengthwise) form to obtain the finished product.

### DESCRIPTION OF DRAWINGS

The utility model is explained further in more detail with reference to the attached drawings (sketches), which show drainages:
Figure 1 - a strip-shaped drainage;
Figure 2 - a drainage in the form of a curved strip;
Figure 3 - a strip-shaped drainage with rounded edges;
Figure 4 - a complex-shaped (T-shaped) drainage;
Figure 5 - a complex-shaped drainage;
Figure 6 - a drainage of a rectangular shape;
Figure 7 - a complex-shaped drainage with a triangle-shaped ledge;
Figure 8 - a complex-shaped drainage with a ledge in the form of a folded rectangle;
Figure 9 - a triangle-shaped drainage with a ledge in the form of a folded rectangle;
Figure 10 - a triangle-shaped drainage with a ledge in the form of (an angle) of a triangle;
Figure 11 - a draining in the form of a square having a perforation with a ledge (in the form of an angle) in the form of a triangle;
Figure 12 - a circle-shaped drainage with a triangle-shaped ledge;
Figure 13 - a drainage in the form of a circle with an aperture;
Figure 14 - a complex-shaped drainage with apertures;
Figure 15 - a drainage in the form of two separate strips;
Figure 16 - an implanted strip-shaped drainage (shown in Figure 1);
Figure 17 - an implanted complex-shaped drainage with a ledge in the form of a folded rectangle (shown in Figure 8);
Figure 18 - an implanted triangle-shaped drainage with a triangle-shaped ledge (shown in Figure 10)

### PREFERRED EMBODIMENTS

As it is shown in the attached drawings (sketches), the drainage according to the application is generally made in the form of a finished flat product made of a bioresorbable material with two end sections.

The preferred or optimal shape and sizes of this type of the drainage can be selected according to the application, taking into account the type and characteristics of the operation performed, the shape and the size of the scleral flap cut out during the surgery intervention, as well as the channel made in the trabecular zone (when using the drainage with a ledge for penetrating operations). This preferred shape of the item is aimed at proving ease of its handling during operations as well as for its most effective functioning after implantation as an antiglaucomatous drainage.

The most preferred drainage sizes are selected from the following ranges:
- a width of 0.5 to 12.0 mm, advantageously 1.0-4.0 mm
- a thickness of 10-600 microns, advantageously 70-150 microns.

Drainages can have a height of 3-20 mm depending on the design, but are not limited to this value. Drainages that have bent parts, including a ledge, and ones described above have a double thickness. The ledge (if any) can have a height of 1.0-4.0 mm, but is not limited to this value, since the angular parts of the drainage structure can be considered as such if it has a shape of a triangle, a square or another.

The optimal shape and size of the drainage depends on the individual characteristics of the patient's eye and the operation technique. Correction of the size and the shape of the drainage, if necessary, can be carried out during the operation by simulation of the drainage.

Moreover, most drainages made in accordance with this application can be installed in different ways. This may concern, for example, a strip-shaped drainage. When installing it there can be a different angle of the drainage outlet of the scleral bed (relative to the base of the flap): into a prilimbal zone of the sclera or over the stacked scleral flap (0⁰), into a prilimbal zone of the sclera, and after bending toward the equator (90°) (Fig. 2) or immediately towards the equator (Fig. 1). Another example of the above is a complex-shaped (T-shaped) drainage. This drainage can be installed with an expanding part - to the equator (Fig. 4) or an expanding part - to the limb, and so that the first end is located on different sides of the flap or wraps around it on both sides.

Synthetic polymers, microbiologically synthesized polymers, and modified natural polymers can be used as bioresorbable materials.

One of the following materials can be used as a synthetic polymer: polyglycolic acid (polyglycolide), D-, L- or D,L-polylactic acid (polylactide), poly-s-caprolactone, polyvinyl acetate, polyvinyl alcohol, copolymers, or block copolymers of the specified polymers or mixtures thereof.

One of the following materials can be used as a microbiologically synthesized polymer: poly-3-hydroxybutyrate, poly-3-hydroxyvalerate, poly-4-hydroxybutyrate, fibrillar protein, copolymers of these polymers or their mixtures.

One of the following materials can be used as a modified natural polymer: ethylcellulose, acetylcellulose (cellulose acetate, diacetate, or triacetate), chitosan, silk, viscose, cellophane, or mixtures thereof.

The set of bioresorbable materials available for use includes, but is not limited to, the above-mentioned materials.

The microfiber drainages described below are made from synthetic polymers and microbiologically synthesized polymers, advantageously hydrophobic. Microfiber drainages made of natural, modified natural polymers and other hydrophilic polymers, as well as composite materials based on them, have been found to have poorly reproducible characteristics, in particular, strain-strength characteristics, as well as the resorption time, due to their hydrophilic properties (a degree of equilibrium swelling in physiological media is more than 15wt.%). For this reason, they are not promising for practical use.

The bioresorbable polymer material from which the device is made according to the application can be continuous and non-continuous, i.e. having pores and / or apertures. The use of the discontinuous material is more preferable, since the pores, apertures and cavities in it provide more effective penetration of intraocular fluid and a greater filtering effect in general, while the structure of the discontinuous material depends on the method of its production. The application considers such options for non-continuous material as a porous film (obtained by washing out a filler), a perforated film, a material obtained by electroforming (a microfiber material), crazing, lithography, bioprinting, as well as a woven material, a mesh, although these options should not be considered as restrictive. It should be noted that such non-continuous materials as a microfiber and a woven also have pores that differ in shape from traditional pores and are determined by the distances between neighboring fibers and threads, respectively.

In general, the drainage under the application is obtained in two main ways. In accordance with the first method, a blank is first obtained, which is a flat sheet of a bioresorbable material from a continuous, porous or perforated film, of a micro-wave or a woven material or a mesh. These blanks can be obtained by casting from a solution or a melt, or a woven from threads or fibers, as shown below. The finished products of an appropriate size and a shape are cut out of these sheets. In accordance with the second method, a blank is first obtained, which is a thin-walled tube made of a bioresorbable material. The resulting blank is cut out in a flat folded or cut (for example, lengthwise) form to obtain the finished product.

A continuous film for obtaining drainages (blanks) is made by a known method when a solvent (organic or aqueous) is evaporated from a pre-prepared polymer solution. This process can be performed at room temperature or by heating. At the same time, thickness of the resulting film is primarily determined by concentration of the original polymer solution.

A porous film for obtaining drainages (blank) is made in a similar way by inserting a subsequently washed out filler into a pre-prepared polymer solution in an organic solvent or into its melt. This inert additive, organic or inorganic, being well soluble in water, after receiving the product is easily and almost completely removed from it after holding in water at a given temperature. The particle size of the additive used and its content (relative to the polymer component) determines, respectively, both the size of the pores formed and the overall porosity of the resulting material. Such an original porous film in accordance with this application can be made either in a flat form (with its subsequent cutting and welding, gluing or wrapping into a product), or immediately in the form of a thin-walled tube.

Perforation of materials obtained from continuous or porous polymer films, in accordance with this application, can be carried out using a special perforator, for example, a dermatome intended for applying through incisions to temporary wound coatings or using a laser intended for laser micro-processing of film materials. The resulting apertures can have a diameter from 20 to 2 000 microns. At the same time, both pre-prepared blanks (continuous film sheets or thin-walled tubes) and ready-made drainages can be subjected to perforation. The first option is preferable because of the greater convenience of fixing the blank.

The drainage (drainage blank) with a non-continuous structure can also be made by other modern and widely described methods: using crazing, electroforming, lithography, and bioprinting. These methods for obtaining non-continuous bioresorbable materials were developed mainly for creating various scaffolds (matrices) populated by cells and intended for cell engineering [T.G. Volova. Materials for medicine, cell and tissue engineering. Krasnoyarsk, SFU PPC. 2009, p. 262].

More specifically non-continuous bioresorbable drainages in accordance with this application can be obtained in the following ways:
- Method of polymer crazing. Polymer crazing is the process of formation of ordered fibrillar-porous structures during orientation extraction of amorphous polymers in adsorption-active liquid media [http://thesaurus.rusnano.com/wiki/article22206]. The resulting nanopores can be filled with any media in which the polymer was stretched, for example, drugs. The resulting material can be attributed to porous or nanoporous films;
- Method of electroforming (electrospinning). Electroforming is a method of pulling fibers from a polymer solution or a melt by applying a high DC voltage, resulting in porous materials with a microfiber structure (with stochastically distributed fibers) [T. Stylianopoulos, C.A. Bashur, A.S. Goldstein et al. Computational predictions of the tensile properties of electrospun fiber meshes: effect of fiber diameter and fiber orientation. J. Mech. Behav. Biomed Mater. 2008, v. 1, Nº4, 326-335]. A diameter of the obtained fibers can be 0.2-20 microns, and porosity of the structure varies from 70 to 95 %. The electroforming unit consists of a perfuser (polymer supply system), a high-voltage power source, and a fixed grounded collector (substrate) [A.Yu. Khomenko. Regulation of morphology and properties of non-woven and highly dispersed biocompatible materials based on chitosan and lactic acid polymers obtained by electroforming. Diss. Ph.D., Moscow, 2016, p. 168; V.N. Vasilets, I.V. Kazbanov, A.E. Efimov, V.I. Sevastyanov. Development of new methods for formation of implantation materials using electrospinning and bioprinting technologies. Bulletin of Transplantology and artificial organs. 2009, v. 11, Nº2, 47-53]. The polymer solution is placed in the perfuser representing a syringe which piston is connected to a stepper motor. In this case, organic compounds with high volatility, such as chloroform or methylene chloride, are usually used as a solvent, which leads to its evaporation already during application of fibers to the substrate. The resulting microfibers are wound on a metal core or a drum. The resulting material can be attributed to microfiber materials;
- Method of lithography [F. Hua, Y. Sun, A. Gaur at al. Polymer Imprint Lithography with Molecular-Scale Resolution. Nano Letters. 2004, v. 4, Nº12, 2467-2471]. The resulting material can be attributed to porous or nanoporous films;
- Method of bioprinting: inkjet (using 3D-inkjet bioprinters), as well as micro-extrusion or laser-mediated bioprinting [http://medach.pro/innovations/bioinzheneriya/3d-bioprinting-of-tissues-and-organs/;
   J. Camp. Development of simple 3D printing scaffold for liver tissue engineering. Thesis. 2002, MIT; Y.Yu, L. Zheng, H.Chen et al. Fabrication of hierarchical polycaprolactone/gel scaffolds via combined 3D bioprinting and electrospinning for tissue engineering. Advances in Manufacturing. 2014, v. 2, Nº3, 231-238]. The resulting material can be attributed to porous or nanoporous films;
- Combined method, for example, hybrid electrospinning - jet bioprinting [http://medach.pro/innovations/bioinzheneriya/3d-bioprinting-of-tissues-and-organs/]. The resulting material can be attributed to porous or nanoporous films.

The microfiber material obtained by electroforming can be characterized by the diameter or the thickness (average) of the formed fibers. In the application drainage, a diameter of fibers should be in the range of 0.1-6.0 microns, advantageously 0.5-4.0 microns.

The microfiber materials used can also be characterized by the pore size. At the same time, the "pores" in them can be divided into two categories: pores that form fibers in a given plane (conventionally, two-dimensional pores) (it is important to consider for the smooth passage of humor) and through pores, conditionally, three-dimensional pores (it is important to consider, for example, for the smooth passage (removal) of agglomerates from dead cells and tissues). The size of "three-dimensional pores" is much smaller than the size of "two dimensional pores" (due to chaotic arrangement of fibers) and the size of the pores is determined by the smallest distance between the fibers forming it.

The pore size and the fiber diameter of the microfiber material is determined using microphotographs taken using optical or electron microscopy.

It was found that the strength characteristics of microfiber materials, in particular, their compressive strength (creasability), sharply deteriorate when the size of through pores exceeds more than 14 microns. This is especially true for materials based on elastic fibers (with an elasticity of more than 10%). At the same time, the material of the antiglaucomatous drainage should not be significantly lost its form under pressure from the surrounding tissues and, especially, at the exit point from under the sutured scleral flap, because this leads to a sharp decrease in its drainage capacity and inevitable hypertension. At the same time, large pores in the drainage material are not needed for the reason that humor drainage is just as effective in presence of small pores (no more than 14 microns), and large cell agglomerates with large sizes do not have time to reduce drainage due to the short resorption period of the application bioresorbable drainage (several months). For this reason, the application drainage made of microfiber material should have through pores preferably no more than 14 microns, advantageously 4-12 microns. Along with them, there may also be pores of larger size.

In addition, it was found that there are certain restrictions for microfiber drainages in shape (when cut out of the blank). Since such drainages are produced by electrospinning, in which the formed fibers are wound on a rotating core of different diameters, such drainages are anisotropic, i.e. their fibers are located mainly unidirectionally along the winding line. When the blank material is subsequently removed from the core in the form of a film, these fibers are mainly directed parallel to its surfaces. When further cutting drainages from the obtained film it is necessary to cut them out so that they were a flat product, and their cross-section preferably had a form of a rectangle, the large sides of which (a drainage width) were the upper and lower sides (a surface) of the above mentioned film (the ratio of its sides, i.e. a width and a thickness of the drainage, is not less than 4:1). When cutting a drainage in the form of a non-continuous material, such as a square or a circular cross-section (a cylindrical or tubular drainage) it will be easily peeled off, that limits or makes it impossible to use it as a drainage.

These cut-out drainages, as well as the original blanks, in accordance with the given production conditions, have through channels, mainly along their plane, and when implanted - in the direction of IOF flow, which, in addition to general porosity, contributes to achieving a hypotensive effect.

The drainage (drainage blank) can also be made by spinning from bioresorbable threads or fibers. As such, numerous surgical resorbed threads and fibers with a thickness of 5-100 microns can be used, which have a high strength and a guaranteed period of biodegradation.

The drainage (drainage blank) can be made directly from bioresorbable threads by pre-winding them on a special form (core) followed by sintering. In this case, a diameter of the used thread can consist 4-250 microns, advantageously 20-40 microns.

A ready-made bioresorbable film, a microfiber, a woven material or a mesh obtained by another method and /or for other purposes can also be used as a drainage blank.

It should be noted that despite the above mentioned optimal sizes of the product, the drainage design makes it possible to obtain this product with individual sizes. It can be taken into account and implemented directly when receiving drainages. For example, in so-called minimally invasive anti-glaucoma operations, the size of the cut flap is 1.5-2 times smaller than in conventional operations, that requires the use of drainages also much smaller than usual. Besides, this can be done by the surgeon himself using simulation during the operation, depending on the size and the shape of the scleral flap formed by him and features of the operation performed. This simulation can be used to reduce the size of the drainage, change the shape of the ledge, or perform other manipulations with the original drainage. Additionally, the surgeon can bend the drainage in the desired area.

Non-penetrating operation using the drainage is preferably performed as follows: first, during the operation, after cutting and bending the scleral flap, basal iridectomy is performed with dissection of a corresponding strip of tissue. After that, the proximal part of the drainage is placed on the scleral bed, under the surface scleral flap, while the second distal part of the drainage is placed on the surface of the sclera, advantageously towards the equator, under the previously separated conjunctiva. Next, the surface flap together with the drainage is fixed to the sclera with one or more sutures (depending on the shape of the separated scleral flap), after which the continuous suture is applied to the conjunctiva. As a drainage it can be inserted not one, but two or more strips under the scleral flap. If there are several separate parts in the second (distal) segment (Fig. 7, 8, 15), pre-separation of the conjunctiva for their placement can be general or performed individually for each separate part (in the form of separate channels). In the latter case, a number of the formed pathways (channels) and, accordingly, the outflow zones of IOF can consist 2 or more.

The penetrating operation using the drainage is preferably performed as follows: a drainage having a ledge is used. First, during the operation, after cutting and bending the scleral flap, basal iridectomy is performed with dissection of a corresponding strip of tissue. After that, the proximal part of the drainage is placed on the scleral bed, under the surface scleral flap in such a way that the triangular ledge is located in the zone of trabeculectomy in a pre-formed channel (inserted into the trabeculectomy aperture) and slightly penetrates into the anterior chamber, and the distal part of the drainage (exiting from under the scleral flap) is placed on the surface of the sclera, advantageously towards the equator, under the previously separated conjunctiva. Next, the surface flap together with the drainage is fixed to the sclera with one or more sutures (depending on the shape of the separated scleral flap), after which the continuous suture is applied to the conjunctiva.

The undoubted advantage of the application drainage is its universality, that is ability of its use in various types and variants of antiglaucomatous operations. Moreover, this can be achieved not only by using different models of drainages described in this application, but also by using one specific model, for example, made in the form of a strip, due to its simulation and the operation technique. It is especially important when the optimal option for performing the specific operation is revealed in its process.

The use of biocompatible materials and their resorption during 1-12 months allow to avoid side reactions, complications (which are aggravated by long and especially constant implantation periods) and the need for re-operations. In this case, the drainage resorption time is determined by its thickness and porosity, as well as the nature of the material used. Thus, the application drainage, having fulfilled its temporary function of stabilizing IOP in the post-surgery period, completely resolves within a certain time, which contributes to creation of the stable outflow pathways of IOP and, accordingly, stabilization of IOP.

Sterilization of manufactured drainages is carried out by radiation or gas method (ethylene oxide) under standard conditions.

Clinical examples of the use of drainages are presented below (examples 20-22). No complications or side reactions were detected when using the described drainages before and after their resorption (with a follow-up period of at least two years).

According to the test results, it was found that the application drainage having a microfiber structure have the best hypotensive effect, is most convenient for operations and, accordingly, is most promising for use as an antiglaucomatous drainage.

It was also found that the application bioresorbable drainage, which have a discontinuous (porous, fibrous and microfiber) structure, is able to sorb significant amounts of various drugs. This is due to both their nature and presence of the well-developed surface.

In this regard, ability of using the application drainage for depositing ex tempore drugs used in ophthalmic surgery is of interest. This technique is convenient for operations, allows to speed up tissue healing and reduce the likelihood of post-surgery complications.

It was found that drainages with short-term holding in solutions or suspensions of drugs (1-20 min) sorb them in an amount of 0.1-20 wt.%. The rate of their sorption and desorption is determined primarily by the nature of the drug.

The application drainage can be used for ex tempore immobilization of the following drugs: steroidal anti-inflammatory drugs (dexamethasone, hydrocortisone, prednisone, triamcinolone, betamethasone, diprospan and others), non-steroidal anti-inflammatory drugs (diclofenac sodium, indomethacin, flurbiprofen and others), cytostatics (5-fluorouracil, mitomycin-C, bleomycin and others) or other types of drugs.

Thus, the application drainage has the following advantages:
- possibility of using the drainage for all forms and types (penetrating and non-penetrating) of antiglaucomatous operations
- use of the drainage promotes the smooth passage of IOF throughout the formed outflow pathways, up to the conjunctival vascularity, including the zone of the active vascular outflow, preventing their splicing, that provides a stable hypotensive effect
- use of biocompatible materials and the optimal time of their resorption allows to avoid side reactions, complications and the need for re-operations
- convenience and ease of installation and ability of simulation during the operation
- possibility of using the drainage for depositing ex tempore drugs used in ophthalmic surgery, that allows to accelerate healing of tissues

### EXAMPLES

### Example 1.

A drainage made of a continuous film. A 3.6 % solution of poly(ε-caprolactone) (reduced viscosity 1.2 dl/g, Corbion, Purac) was prepared in freshly distilled tetrahydrofuran. This solution was poured onto a glass substrate mounted horizontally. After evaporation of the solvent at room temperature, the film on the substrate was dried at 40°C for 60 minutes. After that, to remove the residual solvent, the film was dried in a vacuum drying cabinet at 35°C for 2 hours. Then the blank in the form of a sheet (film) with a thickness of 60 microns was removed from the substrate, after which narrow strips with a width of 5.0 mm were cut out from the blank with a sharp metal cutter. Next, drainages were cut out from this blank in the form of a rectangular strip with sizes 5.0*2.0*0.06 mm (shown in Fig. 1). The drainages were placed in a polypropylene blister and sterilized by the radiation method (fast electrons, 2.0 MPa).

### Example 2.

A 3.2% solution of poly(L-lactide-co-ε-caprolactone) copolymer (85:15, r.viscosity 1.6 dl/g, Corbion, Purac) was prepared in freshly distilled tetrahydrofuran. Then drainage was obtained under the conditions of example 1. A cut-out rectangular strip that had sizes of 14.0*1.0*0.055 mm, was bent in the middle part at a right angle. The drainages were obtained in the form of a curved strip (shown in Fig. 2). The drainages were placed in a polypropylene blister and sterilized by the radiation method (fast electrons, 2.0 MPa).

### Example 3.

A 1.2% solution of poly(D,L-lactide-co-glycolide) copolymer (50:50, r.viscosity 0.62-0.65 dl/g, DURET Corp.) was prepared in freshly distilled dioxane. This solution was poured onto a glass substrate mounted horizontally. To obtain the film, the substrate was heated to 50°C and held at this temperature for 90 minutes. After that, to remove the residual solvent, the film was dried in a vacuum drying cabinet at 50° for 2 hours. Then the blank in the form of a film with a thickness of 12 microns was removed from the substrate, after which drainages were cut out of it in the form of a rectangular strip having sizes 6.0*2.0*0.012 mm. The drainages were placed in a polypropylene blister and sterilized by the radiation method (fast electrons, 2.0 MPa).

### Example 4.

The drainage made of a porous film. In 10.0 g of a 4.0% prepared poly(D,L-lactide) solution (r.viscosity 2.0 dl/g, Corbion, Purac) in freshly distilled chloroform, 2.4 g of finely ground sodium chloride (particle size 20-45 microns) was added and this mixture was thoroughly mixed. Then the resulting suspension was poured on a glass substrate installed horizontally. After evaporation of the solvent at room temperature, the film on the substrate was dried at 30°C for 20 minutes, and then at 40°C for 40 minutes. After that, to remove the residual solvent, the coating was dried in a vacuum drying cabinet at 30°C for 4 hours. Next, the coated substrate was placed into a glass vessel with distilled water and held at a temperature of 60°C for 10 hours until the filler (pore-forming agent) was completely washed out. After that, the coating on the substrate was dried at 80°C for 2 hours, and then in a vacuum drying cabinet at 35°C for 2 hours, after which it was removed as a thin elastic film. Then the drainages in the form of a rectangular strip with rounded edges, having a width of 3.5 mm and a total length of 7.0 mm, were cut out of the resulting blank in the form of a porous film with a thickness of 120 microns (shown in Fig. 3). The prepared drainages were sterilized by gas (ethylene oxide).

### Example 5.

A 4.0% solution of poly(L-lactide) (r.viscosity 1.0 dl/g, Corbion, Purac) was prepared in freshly distilled chloroform, to which 4.2 g of finely ground sucrose (particle size 15-30 microns) was added as a washed out filler. The resulting suspension was poured on a glass substrate installed horizontally. After evaporation of the solvent at room temperature, the film on the substrate was dried at 40°C for 60 minutes. Next, the film was removed from the substrate and placed into a glass vessel with distilled water and held in a drying cabinet at a temperature of 60°C for 10 hours until the filler was completely washed out. After that, the film was dried at 80°C for 2 hours and then in a vacuum drying cabinet at 35°C for 2 hours. Later, the complex-shaped drainages with a width of 4.0 mm and a total length of 12.0 mm were cut out of the blank with a thickness of 110 microns (shown in Fig. 4). The prepared drainages were sterilized by gas (ethylene oxide).

### Example 6.

Under the conditions of example 1, a continuous film drainage was obtained. A 6.0% poly(glycolide) solution (r.viscosity 1.4 dl/g, Corbion, Purac) was prepared in hexafluoroisopropanol. The resulting blank in the form of an elastic film had a thickness of 130 microns. Drainages of a complex shape were cut out of this blank, having a width of 4.0 mm and a total length of 15 mm (shown in Fig. 5).

### Example 7.

Under the conditions of example 1, a drainage was obtained from a continuous film of a composite material. A 5.0% solution of a mixture (80:20) of poly(D,L-lactide-co-glycolide) copolymer (85:15, r.viscosity 0.76-0.85 dl/g, DURET Corp.) and polyvinyl acetate (M_{w}100000, Aldrich) in chloroform was used. The resulting blank in the form of an elastic film had a thickness of 95 microns. Rectangular drainages with a width of 4.0 mm and a total length of 8.0 mm were cut from this blank (shown in Fig. 6).

### Example 8.

Under the conditions of example 1, a drainage with a ledge was obtained from a solid film. A 5.0% solution of a triblock copolymer polylactide (5 000) - polyethylene glycol (10 000) - polylactide (5 000), Polysciences, Inc. in chloroform was used. The resulting blank in the form of an elastic film had a thickness of 90 microns. Drainages of a complex shape were cut out from this blank, having a ledge (from the proximal part) of a triangular shape with a base of 1.0 mm and a height of 1.0 mm. The resulting drainages had a width of 5.0 mm, a total length of 10 mm and a thickness of 85 microns (shown in Fig. 7).

### Example 9.

Under the conditions of example 4, a drainage with a ledge was obtained from a solid film. A 3.0% solution of poly-3-hydroxybutyrate (r.viscosity 0.46 dl/g) in chloroform was used. The resulting pre-blank in the form of an elastic film had a thickness of 100 microns. From this pre-blank, blanks of a complex shape were cut out, having a ledge (from the proximal part) in the form of a rectangle with a base of 2.0 mm and a height of 3.0 mm. Then the ledge was bent along the diagonal of the rectangle and it took the form of a double triangle. The resulting drainages of a complex shape had a width of 10.0 mm, a total length of 18 mm and a thickness of 100 microns (shown in Fig. 8).

### Example 10.

Under the conditions of example 1, a drainage with a ledge was obtained from a solid film. An 8.5% solution of poly-3-hydroxyvalerate (r.viscosity 0.53 dl/g) in chloroform was used. The resulting pre-blank in the form of an elastic film had a thickness of 270 microns. From this pre-blank, blanks in the form of an equilateral triangle with sides of 8.0 mm were cut out, also having a ledge (from the proximal part) in the form of a rectangle with a base of 2.0 mm and a height of 2.0 mm. Then the ledge was bent symmetrically on both sides (angles) and it took the form of a part of a folded envelope. The resulting drainages of a complex shape had a width of 8.0 mm, a total length of 9.0 mm and a thickness of 270 microns (shown in Fig. 9).

### Example 11.

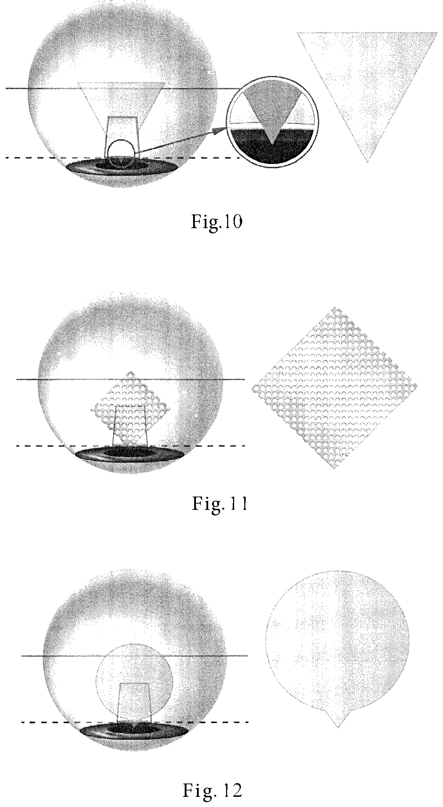

Under the conditions of example 1, a drainage with a ledge was obtained from a solid film. A 3.0% solution of poly-4-hydroxybutyrate (r.viscosity 0.49 dl/g) in chloroform was used. The resulting blank in the form of an elastic film had a thickness of 50 microns. Drainages were cut out in the form of an isosceles triangle from this blank, having a ledge (which was used as the proximal end - a drainage angle). The resulting drainages had a width of 14.0 mm, a total length of 12 mm and a thickness of 50 microns (shown in Fig. 10).

### Example 12.

Under the conditions of example 1, a drainage with a ledge was obtained from a perforated film. A 3.0% solution of poly(3-hydroxybutyrate-co-3-hydroxyvalerate) (r.viscosity 0.58 Dl/g) in chloroform was used. The resulting pre-blank with a thickness of 60 microns was perforated using a http://mehprom-ural.ru/mini-marker-2/ Minimarker 2 fiber laser, NTO "IRE-Polyus", Russian Federation. Apertures with a diameter of 100 microns were made in a staggered order with a step of 300 microns. Next, drainages in the form of a square of 7.0*7.0 mm were cut out of the resulting blank, having a ledge (which was used as the proximal end - a drainage angle). The resulting drainages had a width of 10 mm, a total length of 10 mm and a thickness of 60 microns (shown in Fig. 11).

### Example 13.

Obtaining drainage from a porous film. A solution of fibrillar protein - recombinant spidroin-1 was prepared. To do this, a sample of 0.3 g of recombinant spidroin-1 was dissolved in 4.0 ml of a 10% solution of lithium chloride in 90 % formic acid. 0.9 g of sodium chloride with a particle size of 40-65 microns was added to this solution. Then the resulting suspension was applied to a glass substrate installed horizontally and dried on it at a temperature of 40°C for 3 hours. The resulting film was removed and held in 96% ethanol for 2 hours, in distilled water for another 2 hours, and then dried in a vacuum at 40 °C for 3 hours. The pre-blank was obtained in the form of a porous film with a thickness of 105 microns. Drainages were cut out from this blank in the form of a circle with a diameter of 9.0 mm, having a triangular ledge with a rounded angle with a base of 1.5 mm and a height of 2.0 mm. The resulting drainages had a width of 9 mm, a total length of 11 mm and a thickness of 105 microns (shown in Fig. 12).

### Example 14.

Under the conditions of example 1, a drainage was obtained from a continuous film of a composite material. To a mixture of 30.0 g of a prepared 4.0 % aqueous solution of polyvinyl alcohol (M_{w} 85 000, 96 % hydrolyzed groups, Aldrich) and 5.0 g of a 4.0% aqueous solution of chitosan (low molecular weight food, M 1 000-30 000), CJSC "Bioprogress" it was added 0.35 g of a 25% aqueous solution of glutaric dialdehyde and 0.36 g of glycerol, and then acetic acid to pH 4. The resulting solution was applied to a glass substrate, placed in a drying cabinet and cured at 60°C for 2 hours, and then at 90°C for 1 hour. Next, the resulting film with thickness of 125 microns was removed and ophthalmic treponem with a diameter of 3.0 mm cut a circular aperture, and then treponem with a diameter of 7.0 mm, the middle of which was mounted in the center of the cut aperture, cut the drainage in the form of a circle with an aperture (washers). The resulting drainages had a width of 7.0 mm, a total length of 7.0 mm, and a thickness of 125 mm (shown in Fig. 13).

### Example 15.

Obtaining a drainage from a porous microfiber material by electrospinning (electroforming) [A.Yu. Khomenko. Regulation of morphology and properties of non-woven and highly dispersed biocompatible materials based on chitosan and lactic acid polymers obtained by electroforming. Diss. Ph.D., Moscow, 2016, p. 168; V.N. Vasilets, I.V. Kazbanov, A.E. Efimov, V.I. Sevastyanov. Development of new methods for formation of implantation materials using electrospinning and bioprinting technologies. Bulletin of Transplantology and artificial organs. 2009, v. 11, Nº2, 47-53]. For this purpose, a 4.0% solution of poly-3-hydroxybutyrate (r.viscosity 0.46 dl/g) was used in methylene chloride, which was applied to a brass core with a diameter of 1.6 mm and a length of 10 cm. The speed of the stepper motor was 480 rpm, the polymer feed rate was 0.28-0.35 ml/min, and the voltage was 25 kV. The thickness of the resulting coating is 50 microns. Next, the coated core was placed into distilled water. After that, the resulting tube of a microfiber material was removed from the core and dried at 80°C. After that, it was cut lengthwise, obtaining a flat blank with a thickness of 50 microns, a width of 5.0 mm and a length of 7.0 cm. Next, drainages of complex shape with two apertures were cut out of it, having a width of 5.0 mm, a total length of 12 mm and a thickness of 50 microns, the size of through pores of the material is 3 microns, the average diameter of the fibers is 0.2 microns (shown in Fig. 14).

### Example 16.

Obtaining a drainage from a porous microfiber composite material by electrospinning (electroforming) was performed under the conditions of example 15. A 10.0% solution of a mixture (50:50) of poly-3-hydroxybutyrate (r.viscosity 0.46 dl/g) and a poly(D,L-lactide-co-ε-caprolactone) copolymer (25:75, r.viscosity 0.70-0.90 Dl/g, DURET Corp.) in chloroform was used. A flat blank with a thickness of 190 microns, a width of 5.0 mm and a length of 10.0 cm was obtained, the size of the through pores of the material was 14 microns, and the average fiber diameter was 4.0 microns. Next, drainages were cut out of it, which were two separate strips with dimensions of 10.0*2.0*0.19 mm (shown in Fig. 15).

### Example 17.

Obtaining a drainage from a porous microfiber material) was carried out under the conditions of example 15. A 4.0 % solution of poly-3-hydroxyvalerate (r.viscosity 0.53 dl/g) in chloroform was used. A steel core with a diameter of 8.0 mm and a length of 15 cm was used as a collector. A flat blank with a thickness of 30 microns, the size of the through pores of the material 12 microns, and the average fiber diameter of 1.0 microns were obtained. Next, drainages were cut out of it, which were rectangular strips with sizes of 10.0*1.5*0.03 mm (shown in Fig. 1).

### Example 18.

Obtaining a drainage from a porous microfiber material) was carried out under the conditions of example 15. A 12.0% solution of poly(D,L-lactide-co-glycolide) copolymer was used (50:50, r.viscosity 0.62-0.65 dl/g, DURET Corp.). A steel core with a diameter of 8.0 mm was used as a collector. A flat blank with a thickness of 350 microns, the size of the through pores of the material 8.0 microns, and the average diameter of the fibers 5.0 microns were obtained. Next, T-shaped drainages were cut out of it, having sizes of 12.0*4.0*0.35 mm (shown in Fig. 4).

### Example 19.

Obtaining a drainage from a porous microfiber material having a ledge. The blank was prepared by electroforming with the following parameters: the collector was a steel rod with a diameter of 8.0 mm, the speed of the stepper motor of the installation was 350 rpm, the feed rate of the polymer solution was 1.0 ml/hour, and the voltage was 35 kV. An 8% solution of poly(D,L-lactide-co-glycolide) copolymer (85:15, r.viscosity 0.76-0.85 dl/g, DURET Corp.) in chloroform was used. From the obtained blank, having a thickness of 100 µm, a through pore size of 14 µm, an average fiber diameter of 0.5 µm, complex shapes were cut, having a ledge (from the proximal part) in the form of a rectangle with a base of 2.0 mm and a height of 3.0 mm. Then the ledge was bent along the diagonal of the rectangle and it took the form of a double triangle. The resulting drainages of a complex shape had a width of 10.0 mm, a total length of 18 mm and a thickness of 100 microns (shown in Fig. 8).

### Example 20.

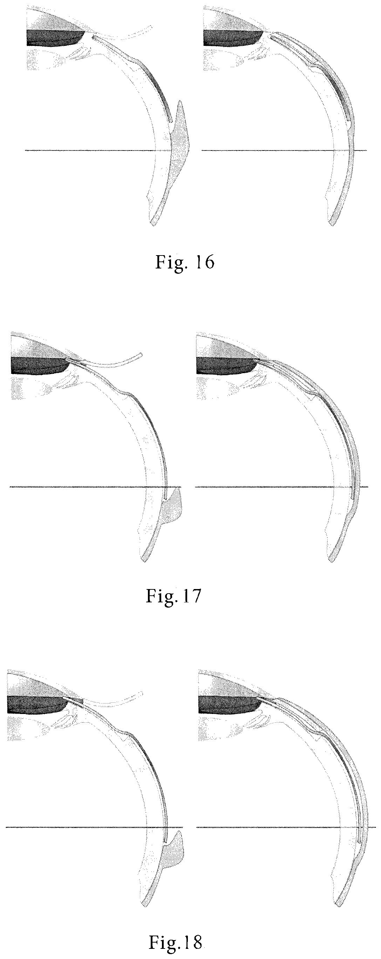

Implantation of a drainage in the form of a strip (shown in Fig. 16).

Clinical example. A patient G., 67 years old, diagnosis OD - primary open-angle laser-operated developed subcompensated glaucoma in the maximum hypotensive mode. OD operation - sinustrabeculectomy with basal iridectomy with implantation of a bioresorbable drainage in the form of a strip (example 1). Processing of the operating field is normal. Anesthesia with Sol. Lidocaini 2% - 2.0 R/b. A suture-holder on the superior rectus muscle. The incision of the conjunctiva at 12 watches to 7 mm from the limb with a length of 6 mm. Hemostasis. From the surface layers of the sclera to a depth of 1/2 of its thickness, a rectangular flap with sides 4 and 3 mm to the light-pervious layers of the cornea is dissected with the base to the cornea. The surface flap of the sclera, together with the conjunctiva, is thrown back on the cornea. In the area of projection of the Schlemm canal, a strip of tissue 1*3 mm is dissected. Basal iridectomy was performed. The bioresorbable drainage is placed on the scleral bed, under the surface scleral flap, so that one half of it is located under the surface scleral flap, and the second half is located on the surface of the sclera. The surface flap together with the drainage is fixed to the sclera by two 8.0 sutures. A continuous suture 8.0 on the conjunctiva. Sol. Dexasoni 0.3 + Sol. Gentamycini 0.2 under the conjunctiva. Aseptic monosticker.

The day after the operation: the eye is almost calm. The front camera is about 2.3 mm (slightly smaller than the average), the humor is light-pervious. There is a spilled drainage filtration above the superior limb. The drainage contour is visible under the conjunctiva of the upper limb. Tn OD 9 mm Hg (pneumatic). 3 months after the surgery operation: OD is calm. There is a spilled filtration pad above the superior limb. The drainage contour is visible under the conjunctiva of the upper limb. Tn OD 13mm Hg. 6 months after the surgery operation, OD is calm. The filtration pad is at 12 watches. The drainage under the conjunctiva is not visible. Tn OD 13mm Hg.

### Example 21.

Implantation of a complex-shaped drainage with a ledge (shown in Fig. 17).

Clinical example. A patient K., 70 years old, diagnosis OS - primary open-angle distant subcompensated glaucoma in the maximum hypotensive mode. OS operation - sinustrabeculectomy with basal iridectomy with implantation of a complex bioresorbable drainage with a ledge. Processing of the operating field is normal. Anesthesia with Sol. Lidocaini 2% - 2.0 R/b. A suture-holder on the superior rectus muscle. The incision of the conjunctiva at 12 watches to 7 mm from the limb with a length of 6 mm. Hemostasis. From the surface layers of the sclera to a depth of 1/2 of its thickness, a rectangular flap with sides 4 and 3 mm to the light-pervious layers of the cornea is dissected. The surface flap of the sclera, together with the conjunctiva, is thrown back on the cornea. In the area of projection of the Schlemm canal, a strip of tissue 1*3 mm is dissected. Basal iridectomy was performed. The bioresorbable drainage is placed on the scleral bed, under the surface scleral flap (example 9) in such a way that the triangular ledge is located in the sinustrabeculectomy zone and partially penetrates into the anterior chamber, and the central strip exits from under the scleral flap to the equator. Two side stripes also exit from under the scleral flap and are located to the left and right of the central one. The surface flap is fixed to the sclera by two 8.0 sutures. Continuous suture 8.0 on the conjunctiva. Sol. Dexasoni 0.3 + Sol. Gentamycini 0.2 under the conjunctiva. Aseptic monosticker.

The day after the operation: the eye is almost calm. The front camera is about 2.5 mm (slightly smaller than the average), the humor is light-pervious. There is a spilled filtration above the superior limb. At 12 watches a triangular drainage ledge is visible paralimbally in the front chamber. The drainage contour is visible under the conjunctiva of the upper limb. Tn OD 7 mm Hg (pneumatic). 3 months after the surgery operation: OD is calm. There is a spilled filtration pad above the superior limb. The drainage contour is visible under the conjunctiva of the upper limb. Tn OD 13 mm Hg. 6 months after the surgery operation, OD is calm. The filtration pad is at 12 watches. The drainage under the conjunctiva is not visible. Tn OD 13 mm Hg.

### Example 22.

Implantation of a drainage in the form of a triangle with a ledge (shown in Fig. 18).

Clinical example. A patient M., 62 years old, OS diagnosis - primary open-angle distant uncompensated glaucoma in the maximum hypotensive mode. OS operation - sinustrabeculectomy with basal iridectomy with implantation of a bioresorbable drainage in the form of a triangle. Processing of the operating field is normal. Anesthesia with Sol. Lidocaini 2% - 2.0 R/b. A suture-holder on the superior rectus muscle. The incision of the conjunctiva at 12 watches to 7 mm from the limb with a length of 6 mm. Hemostasis. From the surface layers of the sclera to a depth of 1/2 of its thickness, a rectangular flap with sides 4 and 3 mm to the light-pervious layers of the cornea is dissected with the base to the cornea. The surface flap of the sclera, together with the conjunctiva, is thrown back on the cornea. In the area of projection of the Schlemm canal, a strip of tissue 1*3 mm is dissected. Basal iridectomy was performed. Bioresorbable drainage (example 11) is placed on the scleral bed under the surface scleral flap in such a way that the angle of the triangle (ledge) is located in the synustrabeculectomy zone and partially, by 0.5 mm, penetrates into the anterior chamber, and the distal end, leaving the scleral flap, is located on the sclera surface. The surface flap together with the drainage is fixed to the sclera by two 8.0 sutures. The protruding ends of the drainage are located on the surface of the sclera, covered with conjunctiva. Continuous suture 8.0 on the conjunctiva. Sol. Dexasoni 0.3 + Sol. Gentamycini 0.2 under the conjunctiva. Aseptic monosticker.

The day after the operation: the eye is almost calm. The front camera is about 2.3 mm (slightly smaller than the average), the humor is light-pervious. There is a spilled filtration above the superior limb. At 12 watches a ledge of the drainage is visible paralimbally in the front chamber. The drainage contour is visible under the conjunctiva of the upper limb. Tn OS 9 mm Hg (pneumatic). 3 months after the surgery operation: OS is calm. There is a spilled filtration pad above the superior limb. The drainage contour is visible under the conjunctiva of the upper limb. Tn OS 12 mm Hg. 6 months after the surgery operation, the patient is calm. The filtration pad is at 12 watches. The drainage under the conjunctiva is not visible. Tn OS 15 mm Hg.

### INDUSTRIAL APPLICABILITY

The utility model can be used in medicine for the surgical treatment of various types of glaucoma (primary, refractory, repeated surgical interventions, in complex cases with secondary glaucoma).

### References

1. E.N. Iomdina, S.M. Bauer, K.E. Kotlyar. Biomechanics of the eye: theoretical aspects and clinical applications. M., Real Time, 2015, p. 208;
2. Biocompatibility. Under the editorship of V.I. Sevastyanov. M., 1999, p. 368;
3. A.V. Tereshchenko, I.A. Molotkova, Yu.A. Bely Yu, E.V. Erokhin. Modification of modern microinvasive non-penetrating glaucoma surgery with the use of the T-shaped drainage. Ophthalmosurgery. 2011, Nº2 [http://www.eyepress.ru/article.aspx?9402];
4. RF Patent Nº2192821, 2002. A method of surgical treatment of severe forms of glaucoma. V.I. Sevastyanov, A.M. Bessmertny, V.P. Erichev at al;
5. S.Yu. Anisimova, S.I. Anisimov, E.V. Larionov at al. Antiglaucomatous "Xenoplast" drainage in non-penetrating and penetrating glaucoma surgery. X Congress of ophthalmologists of Russia. Collection of scientific materials. M., 2015, p. 71;
6. S.Yu. Anisimova, S.I. Anisimov, I.V. Rogacheva. Surgical treatment of refractory glaucoma using a new, biodegradable collagen drainage. Glaucoma. 2006, Nº2, 51-56;
7. RF patent for utility model Nº126931, 2013. Drainage device. A.R. Korigodski, I.B. Alekseev, S.S. Dolgy, A.Yu. Slonimsky;
8. http://www.zdorlife.ru/page-id-512.html;
9. E.A. Egorov, S.N. Basinsky. Clinical lectures on ophthalmology. Training guide. Geotar-Media. 2007, p, 163;
10. T.G. Volova. Materials for medicine, cell and tissue engineering. Krasnoyarsk, SFU PPC. 2009, p. 262;
11. http://thesaurus.rusnano.com/wiki/article22206;
12. T. Stylianopoulos, C.A. Bashur, A.S. Goldstein et al. Computational predictions of the tensile properties of electrospun fiber meshes: effect of fiber diameter and fiber orientation. J. Mech. Behav. Biomed Mater. 2008, v.1, Nº4, 326-335;
13. A.Yu. Khomenko. Regulation of morphology and properties of non-woven and highly dispersed biocompatible materials based on chitosan and lactic acid polymers obtained by electroforming. Diss. Ph.D., M., 2016, p. 168;
14. V.N. Vasilets, I.V. Kazbanov, A.E. Efimov, V.I. Sevastyanov. Development of new methods for formation of implantation materials using electrospinning and bioprinting technologies. Bulletin of Transplantology and artificial organs. 2009, v. 11, Nº2, 47-53;
15. F. Hua, Y. Sun, A. Gaur at al. Polymer Imprint Lithography with Molecular-Scale Resolution. Nano Letters. 2004, v. 4, Nº12, 2467-2471;
16. http://medach.pro/innovations/bioinzheneriya/3d-bioprinting-of-tissues-and-organs/;
17. J. Camp. Development of simple 3D printing scaffold for liver tissue engineering. Thesis. 2002, MIT;
18. Y.Yu, L Zheng, H.Chen et al. Fabrication of hierarchical polycaprolactone/gel scaffolds via combined 3D bioprinting and electrospinning for tissue engineering. Advances in Manufacturing. 2014, v. 2, Nº3, 231-238;
19. http://medach.pro/innovations/bioinzheneriya/3d-bioprinting-of-tissues-and-organs/

## Claims

1. Drainage for antiglaucomatous surgical operations, made in a form of a finished flat product made of a bioresorbable microfiber material, having the first end for at least partial insertion under the separated scleral flap and the second end, made so that it is adapted to be placed in the sclero-conjunctival zone under the separated conjunctiva, while the diameter of the fibers is in the range of 0.1-6.0 microns.

2. Drainage specified in claim 2, **characterized in that** the bioresorbable material is selected from a group that includes: synthetic polymers, microbiologically synthesized polymers or mixtures thereof.

3. Drainage specified in claim 2, **characterized in that** one of the following materials is used as a synthetic polymer: polyglycolic acid (polyglycolide), D-, L- or D,L-polylactic acid (polylactide), poly-s-caprolactone, copolymers or block-copolymers of the specified polymers or their mixtures.

4. Drainage specified in claim 2, **characterized in that** one of the following materials is used as a microbiologically synthesized polymer: poly-3-hydroxybutyrate, poly-3-hydroxyvalerate, poly-4-hydroxybutyrate, copolymers of these polymers or their mixtures.

5. Drainage specified in claim 1, **characterized in that** it has a width of 0.5-12.0 mm and a thickness of 10-600 microns.

6. Drainage specified in claim 1, **characterized in that** it has at least one aperture.

7. Drainage specified in claim 1, **characterized in that** the first end has a ledge facing mainly in the opposite direction from the second end, with ability of introducing the specified ledge into the trabeculectomy zone before its edge exits into the anterior chamber.

8. Drainage specified in claim 7, **characterized in that** the ledge has mainly a shape selected from the following group: a triangle, a rectangle, a trapezoid or specified shapes with rounded or beveled angles or a semi-ellipse.

9. Drainage specified in claim 7, **characterized in that** the drainage ledge is additionally bent in at least one line at a sharp angle to its base.
